# EUROPEAN PATENT APPLICATION

(11) **EP 2 779 001 A2**
(43) Date of publication of application: **17.09.2014**
(21) Application number: 14159047.1
(22) Date of filing: 12.03.2014
(51) Int. Cl.: G06F 19/00

(54) **Daily energy reserve determination**

(30) Priority: 15.03.2013 FI 20135250
(71) Applicant: Laturi Corporation Oy, 90410 Oulu (FI)
(72) Inventor: Tornberg, Vesa, 90410 Oulu (FI)
(74) Representative: Kolster Oy Ab

(57) **Abstract**

There is provided an apparatus for performing the following: acquire test results with respect to a plurality of tests performed by a test person (324); convert the test result of each test into a test score having a common unit, wherein the conversion is based on a test-specific conversion model (400, 500); and determine a daily energy reserve for the test person (324) in time domain on the basis of the plurality of test scores, wherein the daily energy reserve indicates time duration the test person (324) can perform physical and/or mental activities above a predetermined intensity threshold during a day.

## Description

### Field

The invention relates generally to a daily energy reserve of a person. More particularly, the invention relates to determining the daily energy reserve of a test person on the basis of plurality of tests.

### Background

Nowadays it is often discussed that employees are not capable to work intensively for the whole duration of the workday. The working ability has become an increasingly interesting issue to the governmental and commercial entities. Decrease in the overall fitness or energy level generates costs to the society and employers, as the risk of premature retirement or sick leave is increased. Furthermore, limitations in fitness decrease person's performance at work, which inevitably generates costs to the employers. In addition to this, persons most likely desire to perform some after-work activities.

The reason for such incapability to perform activities throughout the day may be that a daily energy reserve of the person is not sufficient enough to provide energy for all the activities during the day. Therefore, there is a need for the employees and other persons to know their daily energy reserves.

### Brief description of the invention

According to an aspect of the invention, there is provided an apparatus as specified in claim 1.

According to an aspect of the invention, there is provided a computer program product as specified in claim 15.

According to an aspect of the invention, there is provided a computer-readable distribution medium carrying the above-mentioned computer program product.

According to an aspect of the invention, there is provided an apparatus comprising means for performing any of the embodiments as described in the appended claims.

Embodiments of the invention are defined in the dependent claims.

### List of drawings

In the following, the invention will be described in greater detail with reference to the embodiments and the accompanying drawings, in which
Figure 1 presents an example curve representing intensity of daily activities with respect to time, according to an embodiment;
Figure 2 shows a method, according to an embodiment;
Figure 3 shows an apparatus, according to an embodiment;
Figures 4 and 5 illustrate example conversion models, according to some embodiments;
Figures 6 and 7A depict methods, according to an embodiment;
Figure 7B illustrates an example of daily energy reserve, according to an embodiment; and
Figures 8A and 8B illustrate a determination of likelihood for working until a predetermined age, according to an embodiment.

### Description of embodiments

The following embodiments are exemplary. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

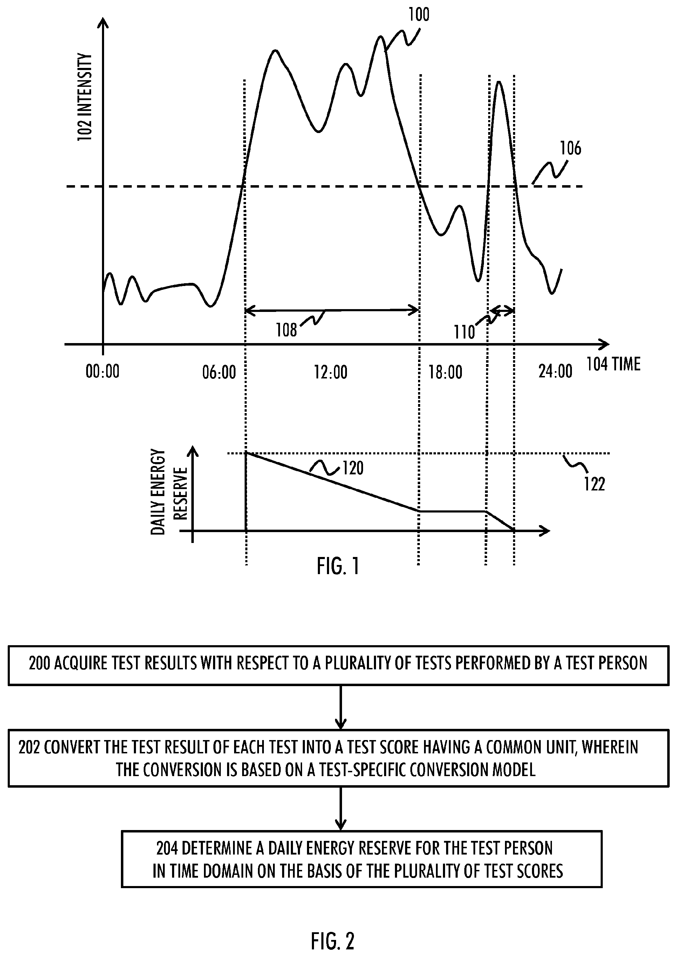

Figure 1 provides an example curve 100 representing intensity of physical and/or mental activities that a person may perform during a given day. In an embodiment, the vertical Y-axis 102 represents a metabolic equivalent of task (MET) value. MET is a physiological measure expressing the energy cost of physical activities and is defined as the ratio of metabolic rate (and therefore the rate of energy consumption) during a specific physical activity to a reference metabolic rate. For example, a MET-value of 1 is equal to the energy produced by an average person seated at rest. As such, one MET is considered as the resting metabolic rate obtained during quiet sitting. For comparison, it may be said that MET values of activities range from 0.9 (sleeping) to 23 (fast running). Alternatively or in addition to, the Y-axis 102 may represent the level of mental activities, such as the level of concentration. In an embodiment, the Y-axis 102 represents a combined measure representing the intensity of physical activities, such as the MET value, and a measure representing the intensity of mental activities. However, in the following, let us consider that the Y-axis 102 represents the MET value. The X-axis 104 represents time of the day.

As shown with the curve 100, the person may be sleeping from approximately 22:00 to 06:00, approximately. As the person wakes up, he/she may start preparing for his/her daily work, for example. During the work, the person's intensity (physical or mental) is typically relatively high and exceeds a certain threshold level 106. The threshold may be a predetermined MET-value, or a predetermined level of mental activity, for example. As the person stops working at approximately 16:00, he/she may rest or perform some lighter activities for a while during which time the depicted physical activity level decreases below the threshold 106. Thereafter, he/she may go jogging, skiing, or perform any other activity that increases the intensity of the physical activities above the threshold 106. After these daily activities, the person may be tired and used all of his/her daily energy level/reserve.

The daily energy level/reserve is shown with the curve 120. As shown with reference numeral 122, when the person wakes up, his/her daily energy reserve may be "full" or at maximum for that person. As the person starts performing physical and/or mental activities and consuming energy, the energy reserve decreases. Although shown in Figure 1 that the energy level drops linearly, this may not be the case, as known by a skilled person. The consumption of energy may be nonlinear, depending on the activities of the person, for example. At the end of the day, the daily energy reserve may be all used up, as assumed in Figure 1. Naturally, there may be days when the person does not perform so intense activities that the daily energy reserve is all used up or alternatively there may be days when the person has performed so intensively that the daily energy reserve has been "over used" or "loaned from the following day".

When the person has sufficient daily energy reserves for performing his/her daily activities, as shown in Figure 1, the person may efficiently contribute to his/her work and also enjoy some important after-work activities without feeling tired or stressed. However, as said, a possible lack in the daily energy reserve of the person may cause problems for the person to work intensively during the workday. This addresses to costs of the employer and of the government. Additionally, limitations in the person's fitness and in overall energy reserve which is available for daily activities may decrease person's overall performance, which lowers general wellness and health level of the person.

Prior art work ability and fitness assessment methods are based on subjective assessment, where medical, occupational health or wellness professional, such as a doctor, inspect the person by using a questionnaire and probably conducts few measurements and drafts a report of the person's ability to work or general fitness status on the basis of the doctors subjective opinion. This method may, however, be time consuming and expensive, especially when a great number of people (e.g. company employees) are being assessed. The result of the assessment may also be very difficult to understand in concrete terms and is always a subjective opinion of the professional inspecting the person.

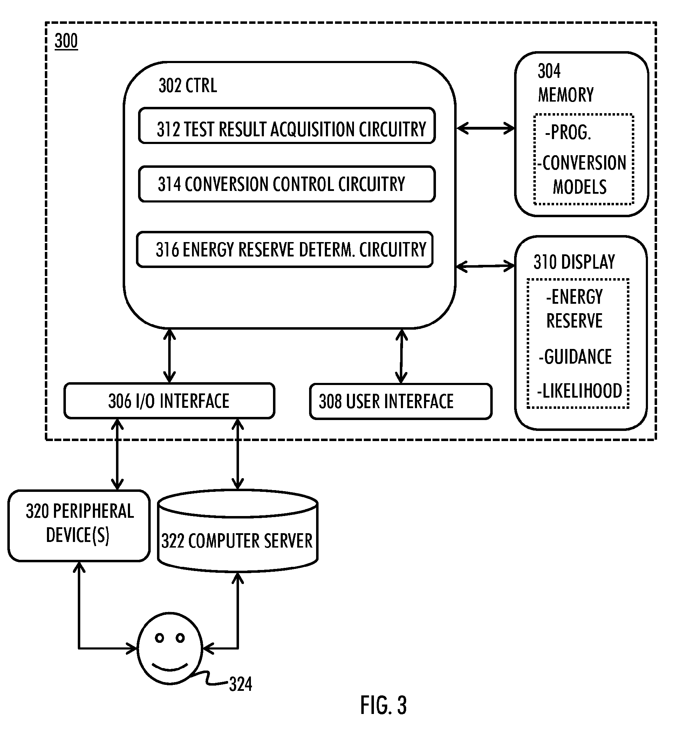

Therefore, it is important to find alternative methods to assess peoples' daily energy reserves and to provide the people with a clear interpretation of the assessment result. Furthermore, guidance to improve or maintain their energy level is advantageously provided. As shown in Figure 2 and 3, there is proposed an apparatus 300 comprising a display 310 for showing information, and at least one processor 302 and at least one memory 304 including a computer program code (PROG). In an embodiment, the apparatus 300 may be or comprise a terminal device, e.g. a user equipment (UE), a user terminal (UT), a smart phone, a personal computer (PC), a wrist unit, a server computer, a laptop, a tabloid computer, a palm computer, or any other communication apparatus.

The at least one memory 304 and the computer program code (PROG) may be configured, with the at least one processor 302, to cause the apparatus 300, and more particularly a test result acquisition circuitry 312, to acquire, in step 200, (primary) test results with respect to a plurality of tests performed by a test person 324. In an embodiment, at least some of the test results may be acquired by the apparatus 300 when the test person 324, or some other person, inputs the test results to the apparatus 300 via a user interface 308. The user interface 308 may comprise, for example, at least one keypad, a microphone, a touch display, a display, a speaker, etc., for allowing the person to control the apparatus 300.

In an embodiment, however, the apparatus 300 comprises an input/output interface 306 comprising hardware and/or software for realizing communication connectivity according to one or more communication techniques, such as wireless local area network (WLAN), Bluetooth, infrared (IR), wired connection, etc. In this embodiment, the apparatus 300 may acquire at least some of the test results from at least one of the following: peripheral sensors 320 connected to the input/output interface 306, a server computer 322 via the input/output interface 306. The peripheral devices 320 may comprise, for example, a training computer, one or more exercise sensors (such as a heart activity monitor), blood pressure monitor, fat percentage monitor, etc. The peripheral devices 320 may be connected to the apparatus 300 via a cable or via a wireless interface, such as the WLAN, Bluetooth or IR, for example.

The computer server 322, such as a computer, laptop, memory storage, etc. may be accessible via a network. Such computer server 322 storing the test results in a cloud may be beneficial as then any person may perform the tests (such as a plurality of physiological tests) at home, for example, and store the test results in a memory of the server 322 accessible by the test person's home computer. The apparatus 300 may then download the test results specific to a test person 324 from the computer server 322, perform some processing, as will be described later, and provide processing results (e.g. the daily energy reserve *Eᵣₑₐₗ*) to the test person's 324 home computer via the network, for example.

In an embodiment the tests performed by the test person 324 comprise a plurality of physiological tests performed by the test person 324. In such case, the acquired test results may indicate at least two of the following with respect to the test person 324: body fat percentage, waist circumference, blood pressure, cardiovascular fitness, muscular strength of upper-body, muscular strength of mid-body, muscular strength of lower-body, flexibility, balance, cholesterol. Each of these may be measured with a corresponding measuring device, by detecting how many repetitions the test person 324 performs with a certain weight or how much weight the test person 324 can lift, for example. The body fat percentage may be measured with a corresponding device, the waist circumference may be measured with a measuring tape, the blood pressure may be measured with a blood pressure sensor, the muscular strength may be measured with detecting repetitions performed in a certain time with a certain weight, for example, the flexibility may be measured with a measuring tape, the balance may be measured with a specific balance board and/or a clock, the cholesterol may be measured with a specific sensor, for example. The cardiovascular fitness may be measured with a heart activity monitor. An example measure for the cardiovascular fitness may be VO₂ max indicating the maximum oxygen uptake, for example. Alternatively, running tests, such as a Cooper test, may be used. In addition to these, height, or weight, body weigh/mass index of the test person 324 may be detected, for example.

In an embodiment, the tests comprise only physiological tests. However, in an embodiment, the apparatus 300 may acquire further test results with respect to a questionnaire presented to the test person. The questionnaire may comprise questions that the test person 324 is to answer. The acquired test results with respect to the questionnaire may indicate at least one of the following with respect to the test person: how stressed the test person feels, how physically vital the test person feels, how tired the test person feels. The questionnaire may thus comprise questions such as how stressed, vital and/or tired the test person feels in a scale of 1 to 10, for example.

As such, the apparatus 300 may acquire the tests results with respect to the plurality of tests. Thereafter, the at least one memory 304 and the computer program code (PROG) may be configured, with the at least one processor 302, to cause the apparatus 300, and more particularly a conversion control circuitry 314, to convert, in step 202, the test result of each test into a test score having a common unit, wherein the conversion is based on a test-specific conversion model. In an embodiment, the unit of each test score is common among all the test scores of all the tests. Having a common (i.e. unitary) unit among all the test scores corresponding to the plurality of test results may be beneficial as then the further processing of the data and obtaining the daily energy reserve is simplified. In an embodiment, the common unit represents or is proportional to time. In an embodiment, the common unit is a minute. In an embodiment, the common unit is the same as the unit used for the daily energy reserve which is to be determined in step 204.

Thereafter, in step 204, the at least one memory 304 and the computer program code (PROG) may be configured, with the at least one processor 302, to cause the apparatus 300, and more particularly a daily energy reserve determination circuitry 316, to determine a daily energy reserve for the test person 324 on the basis of the plurality of test scores. In an embodiment, the daily energy reserve is given in time domain. The daily energy reserve may also be called a real or an actual daily energy reserve/level *Eᵣₑₐₗ*, as opposed to a reference (expected) daily energy reserve *E_{ref}* of an average person. In an embodiment, the daily energy reserve *Eᵣₑₐₗ* indicates time the test person can perform physical and/or mental activities above the predetermined intensity threshold 106 during a day, such as during 24 hours. That is, the daily energy level *Eᵣₑₐₗ* characterizes the time the person can work and be physically active during the day with working intensity that exceed the threshold 106. Looking at Figure 1, such time domain representation of the daily energy reserve may be the sum of periods 108 and 110 during which the person uses all his/her energy reserve of the day, as assumed in the example of Figure 1. It may further be assumed that the person recharges the daily energy reserve while sleeping.

Let us first take a look at how the test-specific conversion models are obtained. In an embodiment, as said, the test results are converted individually into test scores on the basis of a test-specific conversion model. The conversion model applied for a specific test results thus depends on the test corresponding to the specific test result. For example, different conversion models may be applied for converting the test result corresponding to upper-body strength test and for converting the test result corresponding to the cardiovascular fitness test.

Further, in an embodiment, age and gender of the test person 324 may affect the selection of the test-specific conversion model. Thus, the conversion model may be also gender- and/or age-specific, in addition to being test-specific. The age and the gender of the test person 324 may be inputted to the apparatus 300 by the test person 324 or by some other person using the apparatus (such as a person controlling the process for determining the daily energy reserve *Eᵣₑₐₗ*). Alternatively, such information may be acquired from the server computer 322, for example.

In an embodiment, the test-specific conversion model to be applied is based at least partly on a statistical analysis data and/or scientific data on how a given test result affects the time duration the test person can perform physical and/or mental activities above the predetermined intensity threshold 106. The statistical analysis data may thus comprise monitoring a large group of people (i.e. population) and detecting the correspondence of different test results to the daily energy reserve *Eᵣₑₐₗ.* Such statistical and/or scientific data may be found, for example, from at least one of the following documents, which are incorporated herein by reference:
- Gallagher D., Heymsfield S.B., et al.: Healthy percentage body fat ranges: an approach for developing guidelines based on body mass index., American Journal of Clinical Nutrition, 2000, 72, p. 694-701;
- McArdle W.D, et al.: Exercise Physiology: Energy, Nutrition and Human Performance,1991, p. 627;
- McArdle W.D., et al.: Essentials of Exercise Physiology 2nd Edition, 2000, p. 533;
- ACSM's Guidelines for Exercise Testing and Prescription, American College of Sports Medicine, 2006, 7th edition, p. 43-44, 58-61, 84-85;
- Practice Guidelines For Primary Care Physicians: 2003 ESH/ESC Hypertension Guidelines;
- Liikuntatieteellinen seura, kuntotestauksen käsikirja, 2004, p. 276;
- Korhonen O, et al.: Työkuntoprofiili testistön suoritusohjeet 1998, p. 29;
- Liite RY, Kuntotestauksen perusteet 1998, p. 73;
- Viljenen et al.: Strength characteristics of a healthy urban adult population. European Journal of Applied Physiology 1991, 63, p. 43-47;
- Korhonen O, et al.: Työkuntoprofiilitestistön suoritusohjeet, 1998, p. 32;
- Liite Ry, Kuntotestauksen perusteet 1994, p. 146;
- Keskinen KL, et al.: Kuntotestauksen käsikirja: Liikuntatieteellinen seura, 2004, p 182; and
- Suni J, et al.: Fitness for Health: The ALPHA-FIT Test Battery for Adults Aged 18-69. Tester's Manual, UKK-instituutti, 2009, p. 13.

Based on the statistical analysis data found from the above mentioned reference books and other reference material known to a person skilled in the field, it may be detected what the average (i.e. reference) test results for each type of test are. For example, regarding the mid-body strength, which may be measured by the number of sit-ups performed during a predetermined time period, the statistical analysis data may indicate the following table 1. Similar tables may be obtained for each of the plurality of tests from the statistical data available to a skilled person from the above mentioned and other reference documents.

**Table 1: Statistical data for the mid-body strength test (number of sit-ups in a 30 seconds duration)**

| **Male, Age** | **25 - 29** | **30 - 34** | **35 - 39** | **40 - 44** | **45 - 49** | **50 - 54** | **55 - 59** | **60 -** |
|---|---|---|---|---|---|---|---|---|
| **Weak** | ≤ 16 | ≤ 14 | ≤ 12 | ≤ 11 | ≤ 10 | ≤ 9 | ≤ 7 | ≤ 5 |
| **Tolerable** | 17 - 18 | 15 - 17 | 13 - 16 | 12 -15 | 11 - 14 | 10 - 12 | 8 - 11 | 6 - 9 |
| **Average** | 19 - 23 | 18 - 21 | 17 - 19 | 16 - 18 | 15 - 17 | 13 - 16 | 12 - 15 | 10 - 13 |
| **Good** | 24 - 28 | 22 - 26 | 20 - 23 | 19 - 22 | 18 - 21 | 17 - 19 | 16 - 17 | 14 - 15 |
| **Excellent** | ≥ 29 | ≥ 27 | ≥ 24 | ≥ 23 | ≥ 22 | ≥ 20 | ≥ 18 | ≥ 16 |

As shown, a male of 46 years of age performs, on average, between 15 and 17 sit-up repetitions in 30 seconds, for example. As a consequence, in case the test person 324 (of age between 45 and 49 years) performs 16 sit-up repetitions, then it may be considered that the test person mid-body strength is of the average level. Consequently, such test result may not affect an average (or a reference) daily energy reserve *E_{REF}* in any way. However, in case the test person performs only 10 or less repetitions, then it may be considered that the mid-body strength of the test person is weak and this may affect the to-be-determined daily energy reserve *Eᵣₑₐₗ* in a negative manner, as will be described. Thus, it may be detected that a test result below the average level, affects the daily energy reserve *Eᵣₑₐₗ* of the test person 324 in a negative manner and a test result above the average level, affects the daily energy reserve *Eᵣₑₐₗ* of the test person 324 in a positive manner.

As described above, in an embodiment, each test result may have a reference value on the basis of the statistical data, wherein the reference value does not have any effect to the daily energy reserve *Eᵣₑₐₗ* which is to be determined. However, if the test result differs from the reference test result value, then the change causes a change to the daily energy reserve. This reference value may be the average test result for each test and, thus, based on statistical data of a large population. It may be considered that the reference test result corresponds to the reference daily energy reserve *E_{ref}.* That is, if all the test results acquired from the test person 324 correspond to the reference test results, then the daily energy reserve *Eᵣₑₐₗ* may be the same as the reference daily energy reserve *E_{ref}.* The reference daily energy reserve *E_{ref}* may be based on statistical data and on detection that, on average, a person has energy to perform activities above the predetermined intensity threshold 106 for up to 8 hours, for example.

The amount of negative or positive effect each negative or positive test result has on the daily energy reserve *Eᵣₑₐₗ* may be based on the statistical analysis data. Accordingly, based on the statistical data, the conversion models may be predetermined for each test so as to define the amount of effect each test result has to the daily energy reserve *Eᵣₑₐₗ.*

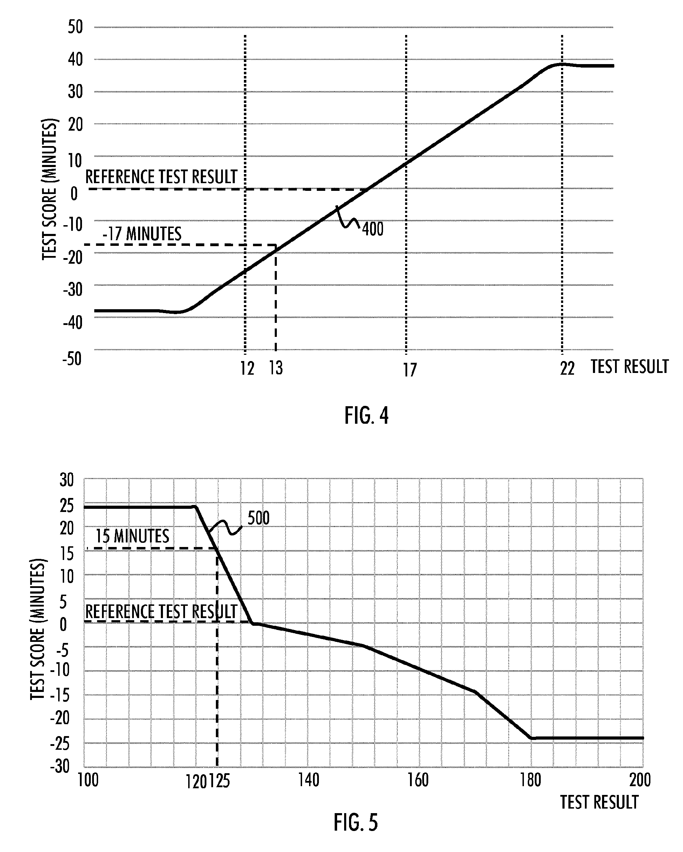

Let us take a look at an example test-specific conversion model by referring to Figure 4, which depicts the conversion model 400 for the mid-body strength test of a male between 45 and 49 years of age. In this example, the unit of the test score shown on Y-axis is a minute. As shown, the average (reference) test result value of 16 repetitions corresponds to a test score of 0 minute. Whereas a low test result (lower than the average test result 16) corresponds to a negative minute reading, a high test result (higher than the average test result 16) corresponds to a positive minute reading. In a similar manner conversion models may be acquired for each test, for each gender and for each age range.

Some of the test-specific conversion models are of linear nature (such as the one shown in Figure 4). However, some of the test results may be of non-linear nature due to the type of the test and on the basis of how a given test result affects the daily energy reserve *Eᵣₑₐₗ.* For example, it may be that a decrease of the test result from the average value does not cause as significant change to the daily energy reserve *Eᵣₑₐₗ* as the same size of decrease of the test result from an already decreased value. In an embodiment, it may also be that a decrease of the test result causes a proportionally different change to the daily energy reserve *Eᵣₑₐₗ* than an increase of the same amount. For example, it has been determined that the conversion model for a blood pressure test may not be linear. Table 2 shows statistical analysis data for the blood pressure tests performed to a plurality of people. For example, it may be seen that the blood pressure is considered slightly increased when the systolic blood pressure is between 131 mmHg and 140 mmHg.

**Table 2: Statistical data for the blood pressure test**

| **Blood Pressure** | **Systolic** | **Diastolic** |
|---|---|---|
| **Optimal** | ≤120 | ≤80 |
| **Normal** | 121 - 130 | 81 -85 |
| **Slightly increased** | 131 - 140 | 86 - 90 |
| **Increased** | 141 - 160 | 90 - 100 |
| **High** | 160 - 180 | 100 - 110 |
| **Very high** | >180 | >110 |

Regarding the blood pressure test, it may have been detected from the statistical data that a change of the test result from 135 mmHg to 145 mmHg is not as significant to the person's health and energy reserve as a change from 145 mmHg to 155mmHg, for example. Further, it may have been detected that the risk of cardiovascular diseases increases significantly as the diastolic blood pressure increases by 20 mmHg or the systolic pressure increases by 10 10 mmHg. At least partly on the basis of such detections, the conversion model for the blood pressure test result may be depicted as shown in Figure 5. In this example, the unit of the test score shown on Y-axis is a minute. The horizontal X-axis depicts the measured systolic blood pressure of the test person 324. As shown, the conversion model 500 is not linear throughout the test result range.

The shape of the conversion curve 400, 500, such as an angular coefficient, may be predetermined on the basis of the statistical and/or scientific data and, more particularly, detection on how the result of a given test affects the daily energy reserve *Eᵣₑₐₗ* determination. The memory 304 may store the plurality of predetermined test-specific conversion models. In general it may be said that when the test result is in an optimal area or in a "better than average" -area, the test score may reflect increased effect to the daily energy reserve *Eᵣₑₐₗ.* It should be noted that the "better than average" -area may be above the average (e.g. in case of cardiovascular fitness) or below the average level (e.g. in case of blood pressure) depending on the type of the test. When the test result is in a "worse than average" -area, the test score may reflect decreased effect to the daily energy reserve *Eᵣₑₐₗ* determination.

The test results of the questionnaire may also be converted into test scores. For example, the more tired the test person 324 feels the more negative effect the test score has on the daily energy reserve determination. The shape of the conversion model may also for these tests be based on statistical and/or scientific data. In an embodiment, the test results of the questionnaire are combined into one test result. In another embodiment, each test result corresponding to the questions of the questionnaire are converted individually by applying test specific conversion models.

As said, the effect of the result of a given test to the energy reserve, which is represented by the test score, may be based on the empirical analysis and/or scientific data available. Additionally, each test may be advantageously weighted and the weight factors may be used for modifying the conversion model. It may be, for example, that for some specific type of occupation, the upper-body strength may be relatively more important than the balance, for example. Therefore, the apparatus 300 may be caused in step 600 to acquire knowledge of weight factors for each test performed to the test person 324. Each weight factor may be based on at least one of the following: the type of the test, the occupation of the test person 324. Further, the selection of the weight factors may be based on the statistical data. For example, if it is detected, on the basis of the statistical data, that a low/high test result in a specific test is more significant than in some other test(s), then that specific test may be weighted more heavily. Table 3 shows non-limiting example weighting of different tests for different types of occupations.

**Table 3: Weigh factors for different tests**

| **Test \ Work type** | **Weight (%) in sedentary work type (e.g. office)** | **Max increase or decrease (minutes)** | **Weight (%) in varied work type (e.g. store, transp.)** | **Max increase or decrease (minutes)** | **Weight (%) in active work type (e.g. manufact.)** | **Max increase or decrease (minutes)** |
|---|---|---|---|---|---|---|
| **1. Fat percentage** | 10 | 24 | 10 | 24 | 10 | 24 |
| **2. Waist circumference** | 10 | 24 | 10 | 24 | 10 | 24 |
| **3. Blood pressure: SYS** | 10 | 24 | 10 | 24 | 10 | 24 |
| **4. Blood pressure: DIA** | 10 | 24 | 10 | 24 | 10 | 24 |
| **5. Cardiovascular fitness** | 15 | 36 | 13 | 31,2 | 11 | 26,4 |
| **6. Strength, mid body** | 15 | 36 | 16 | 38,4 | 15 | 36 |
| **7. Strength, upper limbs** | 5 | 12 | 6 | 14,4 | 7 | 16,8 |
| **8. Strength, lower limbs** | 5 | 12 | 6 | 14,4 | 7 | 16,8 |
| **9. Flexibility** | 5 | 12 | 5 | 12 | 5 | 12 |
| **10. Balance** | 3 | 7,2 | 4 | 9,6 | 5 | 12 |
| **11. Your current vitality level (questionnaire)** | 6 | 14,4 | 5 | 12 | 5 | 12 |
| **12.Your current stress level (questionnaire)** | 6 | 14,4 | 5 | 12 | 5 | 12 |
| **Total** | **100%** | **240 min.** | **100%** | **240 min.** | **100%** | **240 min.** |

As seen from Table 3, in this non-limiting example embodiment, the range of values for the daily energy reserve *Eᵣₑₐₗ* is limited according to predetermined limits. In this example case, the maximum and/or minimum change from the reference daily energy reserve *E_{ref}* is limited to 240 minutes, that is, to 4 hours. It should be noted that other values are possible as well, such as 6 or 8 hours, to mention only a few non-limiting example values. In Table 3, considering that the reference daily energy reserve *E_{ref}* is 8 hours, then the maximum value determined for any test person is 12 hours and the minimum value is 4 hours. The minimum and maximum values shown in Table 3 are only example, non-limiting values.

As shown, in an embodiment, the test corresponding to the cardiovascular fitness may be considered important on the basis of statistical and scientific data. For example, it may have been detected that the cardiovascular fitness affects the most in the daily energy reserve of any person. Therefore, the predetermined weight factor for the cardiovascular fitness test is, in this non-limiting example, 15 percent for a sedentary work type. The exact amount of percentages may be predetermined for each test and for each work type so as to reflect the type of the work, for example. The weight factor of 15 percent causes the maximum and/or minimum effect the test result has for the daily energy reserve *Eᵣₑₐₗ* determination to be 36 minutes (=0,15*240min). For the active work type, the cardiovascular fitness may still be the most important but the weight factors for the strength-related tests may be increased. This may be because the strength of the person may be more important in active work than in sedentary work. As is clear from above, at least partly on the basis of the weight factors, the apparatus 300 may in step 602 determine a maximum and/or a minimum test score value for each test and for each type of occupation.

In an embodiment, for one test person 324, the determined daily energy reserve may be different depending on what the occupation of the test person 324 is. This may be beneficial as then the daily energy reserve *Eᵣₑₐₗ* may reflect the work type of the test person 324. It should be noted that in different works the same test person 324 may use all of his/her energy in different time durations, depending on the physiological fitness of the test person 324. For example, a strong person with a low cardiovascular fitness may perform activities longer in an active work than in a sedentary work.

In an embodiment, in step 604, the test-specific conversion models 400, 500 may be modified on the basis of the determined maximum and minimum values. This may be why the test-specific conversion models 400 and 500 depicted in Figures 4 and 5 are "cut" so as to represent a horizontal line at the edge parts of the models 400 and 500. Such horizontal line represents the maximum effect and/or the minimum effect that each physiological test is allowed to have to the daily energy reserve *Eᵣₑₐₗ* determination. It should be noted though that Figures 4 and 5 are example figures and do not correspond to Table 3 directly. Further, it may be noted that, in an embodiment, the maximum and the minimum values are used in limiting the test scores after obtaining the test scores from the conversion models. Thus, in this embodiment, the generation of the conversion models 400, 500 may be simplified as no limitations need to be posed to the conversion models 400, 500. For example, if the detected test score for the cardiovascular fitness shows 50 minutes positive effect to the daily energy reserve determination, then the test score may be adjusted into 36 minutes, i.e. to the maximum effect the cardiovascular test is allowed to have to the daily energy reserve determination.

In an embodiment, the effect of at least one test result to the daily energy reserve *Eᵣₑₐₗ* is increased when a predetermined criterion is met. For example, if the fat percentage is not obtained, then the effect of the waist circumference test result may be, for example, doubled. Similarly, if the test result for the upper-body strength is not obtained, the effect that the test result corresponding to the mid- or lower-body strength has on the daily energy reserve determination may be, for example, doubled.

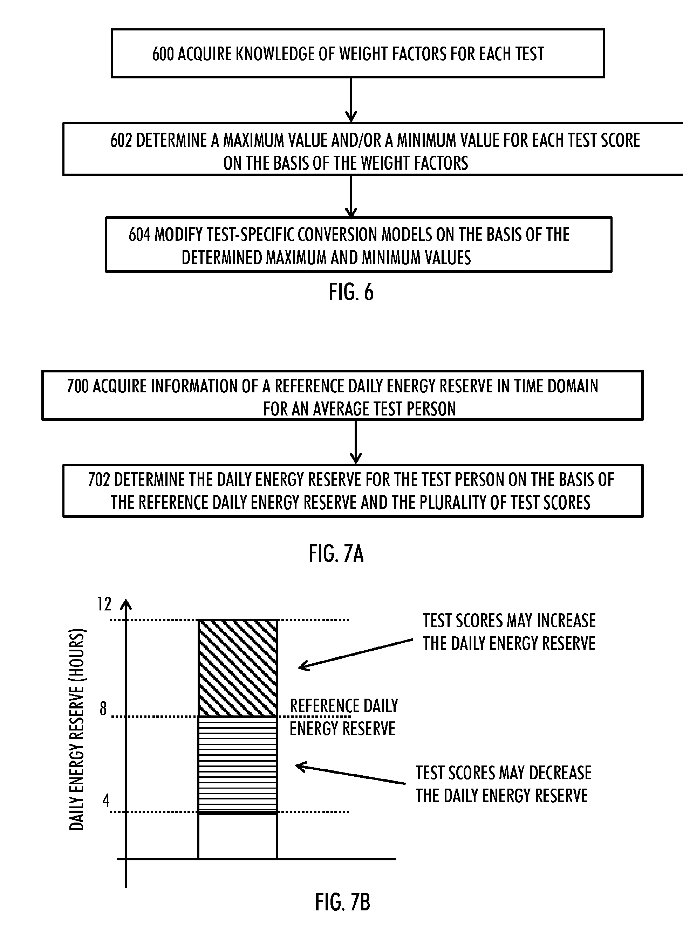

Thereafter, as the test scores corresponding to the test results are obtained by applying the conversion models, the process may proceed to the step 204 in which the daily energy reserve *Eᵣₑₐₗ* for the test person 324 is determined on the basis of the plurality of test scores. Let us look at this further, by referring to Figures 7A and 7B, wherein in step 700, the apparatus 300 is caused to acquire information of a reference daily energy reserve *E_{ref}* in time domain for an average test person. This reference daily energy reserve *E_{ref}* may be predetermined to be, for example, 8 hours, that is, 480 minutes, as shown in Figure 7B.

In step 702, the apparatus 300 may be caused to determine the daily energy reserve *Eᵣₑₐₗ* for the test person 324 on the basis of the reference daily energy reserve *E_{ref}* and the plurality of test scores. This may comprise, for example, adjusting the reference daily energy reserve *E_{ref}* on the basis of the plurality of test scores For example when the unit of each test score is a minute, then the apparatus 300 may deduct or sum each test score from the reference daily energy reserve *E_{ref}* of 480 minutes, as shown in Figure 7B. Figure 7B assumes that the maximum and/or minimum change from the reference daily energy reserve *E_{ref}* is 240 minutes, that is, 4 hours. Thus, assuming that the expected, reference daily energy reserve *E_{ref}* is 8 hours, then the maximum value determined for any test person is 12 hours and the minimum value is 4 hours. However, also here it should be noted that the minimum and maximum values are only example, non-limiting values. A skilled person would readily understand that some other values may be selected as the minimum and maximum values. This way, some of the test scores may affect the daily energy reserve determination in a positive manner (i.e. increases the daily energy reserve *Eᵣₑₐₗ*) whereas some test scores may affect the daily energy reserve determination in a negative manner (i.e. decreases the daily energy reserve *Eᵣₑₐₗ*). Further, some test scores corresponding to the reference test results may not affect the daily energy reserve determination at all. For example, the test score corresponding to the sit-up test may cause the daily energy reserve *Eᵣₑₐₗ* to be approximately 17 minutes lower than the reference daily energy reserve *E_{ref}* assuming that the test person 324 makes 13 sit-ups, as shown in Figure 4. On the other hand, the daily energy reserve *Eᵣₑₐₗ* may be increased by 15 minutes if the test person 324 has a systolic blood pressure of 125 mmHg, as shown in Figure 5. Therefore, on the basis of these two tests only, the actual daily energy reserve *Eᵣₑₐₗ* would be 480-17+15=478 minutes.

In an embodiment, the apparatus 300 is caused to acquire information of the reference values for each test result. Looking at Figure 4, for the sit-up test performed by a male between 45 and 49 years of age, the reference test result may be 16 sit-ups, which corresponds to 0 minutes when converted into the test score. For the systolic blood pressure test shown in Figure 5, the reference test result may be 130 mmHg, which corresponds to 0 minutes when converted into the test score.

In an embodiment, the step of determining the daily energy reserve for the test person 324 comprises taking an average value of the test scores. Then the average test score may be deducted or added to the reference daily energy reserve *E_{ref}* in order to obtain the actual or real daily energy reserve for the test person.

As shown, in some embodiments, the test score represents a positive or a negative value which is to be added to the reference daily energy reserve *E_{ref}* in order to obtain the real daily energy reserve *Eᵣₑₐₗ* for the test person 324. In another embodiment, each test score represents a test-specific daily energy reserve *Eᵣₑₐₗ^{T}.* For example, in Figure 4, the range of values on the vertical Y-axis would then run from 430 minutes to 530 minutes, instead of running from -50 to 50 minutes. In such case, the step of determining the daily energy reserve *Eᵣₑₐₗ* for the test person 324 comprises taking the average value of the test scores. This may be provide ease of implementation as then the acquired test scores need not be added/deducted from the reference daily energy reserve *E_{ref}.*

Let us then take a look on an example test results from the test person 324 as shown in Table 4 and how they are used for determining the daily energy reserve *Eᵣₑₐₗ*

**Table 4: Determination of the daily energy reserve on the basis of the plurality of test scores**

| **Test type** | **Test result** | **Test score** |
|---|---|---|
| **a. Perceived ability (questionnaire)** | 15 | 8 h, 20 min (=+20 min) |
| **b. Body Composition (fat %)** | 22 % | 7h, 58 min (=-2 min) |
| **c. Waist circumference** | 92 cm | 8 h, 8 min (=+8 min) |
| **d. Blood pressure (systolic & diastolic)** | 130/90 | 7 h, 53 min (=-7 min) |
| **e. Cardiovascular fitness (e.g. VO2max)** | 44 mm/l | 8 h, 42 min (=+42 min) |
| **f. Muscular strength, upper-body** | 14 reps. | 7 h, 55 min (=-5 min) |
| **g. Muscular strength, mid-body** | 16 reps. | 8 h, 0 min (=0 min) |
| **h. Muscular strength, lower-body** | 20 reps. | 8 h, 4 min (=4 min) |
| **i. Flexibility** | 22 cm | 7 h, 51 min (=-9 min) |
| **j. Balance** | 52 sec. | 8 h, 0 min (=0 min) |
| **Total score (=Daily Energy Reserve)** | | **8 h, 51 min** |

As shown, the determined daily energy reserve *Eᵣₑₐₗ* may be the average of the test scores. The test score values are obtained from the test-specific conversion models. The conversion models, on the other hand, are derived from the statistical data corresponding to a large population, as explained.

In an embodiment, the determined daily energy reserve *Eᵣₑₐₗ* indicates aggregated time duration the test person can perform physical and/or mental activities above the predetermined intensity threshold 106 during a day. Thus, looking at Figure 1, the aggregate time duration may be the sum of periods 108 and 110, not necessarily one continuous time period.

In an embodiment, the at least one memory 304 and the computer program code (PROG) may be configured, with the at least one processor 302, to cause the apparatus 300 to display the daily energy reserve *Eᵣₑₐₗ* on the display 310, as shown in Figure 3.

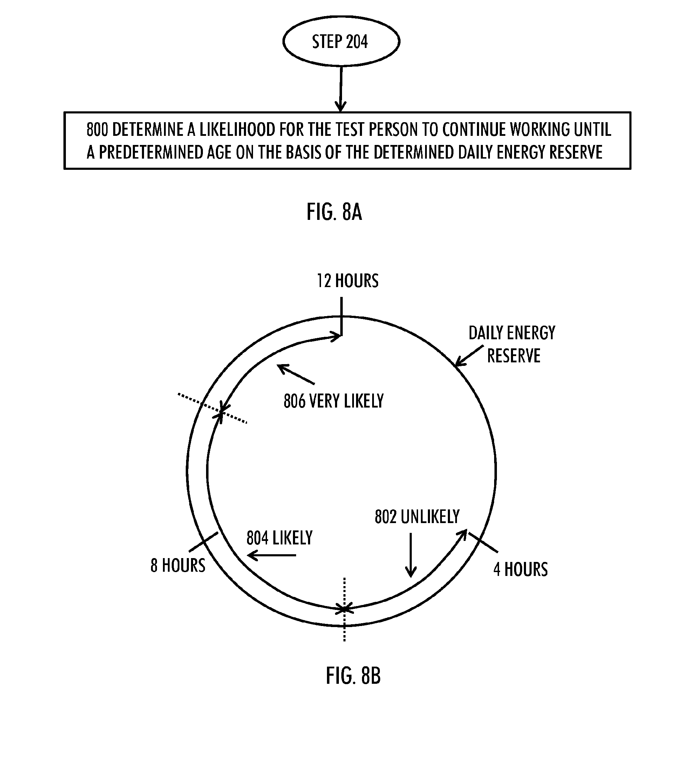

In an embodiment, the overall test score (=daily energy reserve *Eᵣₑₐₗ*) characterizes the person's long-term fitness and health trend. The overall test score may represent a probability that the person's fitness and health level remains above a predefined level for the remaining work career, e.g. until to target pension age of the person. Therefore, in an embodiment, as shown in Figures 8A and 8B, the apparatus 300 is further caused to determine, in step 800, a likelihood for the test person 324 to continue working until a predetermined age on the basis of the determined daily energy reserve *Eᵣₑₐₗ.* The predetermined age may be, for example, a retirement age in that country or profession. An example age may be 63 years, for example. For example, as shown in Figure 8B, when the determined daily energy reserve *Eᵣₑₐₗ* is within the range 802 marked with the bidirectional arrow, it may be determined that it is unlikely that the test person 324 may continue working or be fit to work until the retirement age. On the other hand, when the determined daily energy reserve *Eᵣₑₐₗ* is within the range 804 or within the range 806, it may be determined that it is likely or very likely, respectively, that the test person 324 may continue working until the predetermined age. The boundary values of ranges 802 to 806 may be acquired on the basis of the statistical data of a large population. Figure 8B shows only an example, non-limiting values.

In an embodiment, the determined likelihood or probability is given in verbal indications, whereas in another embodiment, the likelihood is given in numeric probability value, e.g., as a percentage value. In an embodiment, the likelihood is displayed on the display 310, as shown in Figure 3.

In an embodiment, the apparatus 300 may be further caused to display each of the plurality of test results on the display 310. Thereafter, the apparatus 300 may acquire instructions for selecting a set of at least one test result for the determination of the daily energy reserve *Eᵣₑₐₗ.* The instructions may be given by the test person 324 or another user of the apparatus 300 via the user interface 308, for example. The instructions may indicate the apparatus 300 to use only certain test results for the determination of the daily energy reserve *Eᵣₑₐₗ.*

In another embodiment, the apparatus 300 may acquire instructions for adjusting at least one of the test result values in order to detect how the change affects the daily energy reserve *Eᵣₑₐₗ.* The user 324 may apply the user interface 308 in giving these instructions. Such adjusting may be beneficial as then the user 324 may instantly see how the daily energy reserve *Eᵣₑₐₗ* may be improved by changing at least one of the test results. For example, when the test person 324 detects that an improvement in the cardiovascular fitness increases the daily energy reserve *Eᵣₑₐₗ,* the test person 324 may be more eager to start training in order to improve the cardiovascular fitness of the test person 324.

In an embodiment, the apparatus 300 may generate a personal health guidance based on the determined daily energy reserve *Eᵣₑₐₗ* and/or at least one of the plurality of test scores. The health guidance may comprise indicating which test result(s) should be improved in order to improve the daily energy reserve *Eᵣₑₐₗ.* For different daily energy reserves, there may be a pre-described personal health guidance stored in the memory 304. The personal health guidance may indicate how the test result may be improved. For example, if it is detected that the cardiovascular fitness is to be increased, then the apparatus 300 may indicate that the test person 324 is to perform physical activities below the aerobic threshold at least three times per week, for example. The indicated health guidance may be based on sports and health related reference documentation, for example. In an embodiment, the apparatus 300 may display the personal health guidance on the display 310, as shown in Figure 3. The personal health guidance may be reported to the test person 324 in text, numeric and/or visual format by the apparatus 300.

In an embodiment, the test person 324 may repeat the daily energy reserve determination process according to Figure 2 periodically. For example, after the test person 324 has performed some training for improving at least one of the test results, the test person 324 may repeat the process so as to see if his/her daily energy reserve *Eᵣₑₐₗ* has increased or not.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof. For firmware or software, the implementation can be carried out through modules of at least one chip set (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, etc., described with regard thereto, and they are not limited to the precise configurations set forth in the given figures, as will be appreciated by one skilled in the art.

Embodiments as described may also be carried out in the form of a computer process defined by a computer program. The computer program may be in source code form, object code form, or in some intermediate form, and it may be stored in some sort of carrier, which may be any entity or device capable of carrying the program. For example, the computer program may be stored on a computer program distribution medium readable by a computer or a processor. The computer program medium may be, for example but not limited to, a record medium, computer memory, read-only memory, electrical carrier signal, telecommunications signal, and software distribution package, for example. Coding of software for carrying out the embodiments as shown and described is well within the scope of a person of ordinary skill in the art.

Even though the invention has been described above with reference to an example according to the accompanying drawings, it is clear that the invention is not restricted thereto but can be modified in several ways within the scope of the appended claims. Therefore, all words and expressions should be interpreted broadly and they are intended to illustrate, not to restrict, the embodiment. It will be obvious to a person skilled in the art that, as technology advances, the inventive concept can be implemented in various ways. Further, it is clear to a person skilled in the art that the described embodiments may, but are not required to, be combined with other embodiments in various ways.

## Claims

1. An apparatus, comprising:
at least one processor (302) and at least one memory (304) including a computer program code, wherein the at least one memory (304) and the computer program code are configured, with the at least one processor (302), to cause the apparatus at least to:
acquire (200) test results with respect to a plurality of tests performed by a test person (324);
convert (202) the test result of each test into a test score having a common unit, wherein the conversion is based on a test-specific conversion model (400, 500); and
determine (204) a daily energy reserve for the test person (324) in time domain on the basis of the plurality of test scores, wherein the daily energy reserve indicates time duration the test person (324) can perform physical and/or mental activities above a predetermined intensity threshold during a day.

2. The apparatus of claim 1, wherein the apparatus further comprises an input/output interface (306), and wherein the at least one memory and the computer program code are configured, with the at least one processor (302), to cause the apparatus further to:
acquire at least some of the test results from at least one of the following: peripheral sensors (320) connected to the input/output interface (306), a server computer (322) via the input/output interface (306).

3. The apparatus of any of claims 1 to 2, wherein the tests comprise a plurality of physiological tests performed to the test person (324), and the acquired test results indicate at least two of the following with respect to the test person (324): body fat percentage, waist circumference, body mass index, blood pressure, cardiovascular fitness, muscular strength of upper body, muscular strength of mid body, muscular strength of lower body, flexibility, balance, cholesterol.

4. The apparatus of any of claims 1 to 3, wherein the unit of each test score is common among the test scores, and the common unit of the test score is or is proportional to a time domain unit.

5. The apparatus of any of claims 1 to 4, wherein the test-specific conversion model (400, 500) is based at least partly on a statistical analysis on how a given test result affects the time duration the test person (324) can perform physical and/or mental activities above the predetermined intensity threshold.

6. The apparatus of any of claims 1 to 5, wherein the at least one memory and the computer program code are configured, with the at least one processor (302), to cause the apparatus further to:
acquire (600) information of weight factors for each test, wherein each weight factor is based on of at least one of the following: the type of the test, the occupation of the test person (324); and
determine (602) a maximum and/or a minimum test score value for each test at least partly on the basis of the weight factors.

7. The apparatus of claim 6, wherein the at least one memory and the computer program code are configured, with the at least one processor (302), to cause the apparatus further to:
modify (604) the test-specific conversion models (400, 500) on the basis of the determined maximum and minimum values.

8. The apparatus of any of claims 1 to 7, wherein the at least one memory and the computer program code are configured, with the at least one processor (302), to cause the apparatus further to:
acquire information of a reference test result value for each test, wherein the reference test result values do not have any effect to the determined daily energy reserve.

9. The apparatus of any of claims 1 to 8, wherein the at least one memory and the computer program code are configured, with the at least one processor (302), to cause the apparatus further to:
acquire (700) information of a reference daily energy reserve for an average test person (324); and
determine (702) the daily energy reserve for the test person (324) on the basis of the reference daily energy reserve and the plurality of test scores.

10. The apparatus of any of claims 1 to 9, wherein the step of determining the daily energy reserve for the test person (324) comprises taking an average value of the test scores.

11. The apparatus of any of claims 1 to 10, wherein the predetermined intensity threshold corresponds to a certain metabolic equivalent of task, MET, value of the test person (324).

12. The apparatus of any of claims 1 to 11, wherein the at least one memory and the computer program code are configured, with the at least one processor (302), to cause the apparatus further to:
determine (800) a likelihood for the test person (324) to continue working or be fit to work until a predetermined age on the basis of the determined daily energy reserve.

13. The apparatus of any of claims 1 to 12, wherein the at least one memory and the computer program code are configured, with the at least one processor (302), to cause the apparatus further to:
display each of the plurality of test results on a display (310) of the apparatus; and
acquire instructions for performing at least one of the following: selecting a set of at least one test result for the determination of the daily energy reserve, and adjusting at least one test result value.

14. The apparatus of any of claims 1 to 13, wherein the at least one memory and the computer program code are configured, with the at least one processor (302), to cause the apparatus further to:
generate a personal health guidance based on the determined daily energy reserve and/or at least one of the plurality of test scores; and
display the personal health guidance on the display (310) of the apparatus.

15. A computer program product embodied on a distribution medium readable by a computer and comprising program instructions which, when loaded into an apparatus, cause the apparatus at least to:
acquire (200) test results with respect to a plurality of tests performed by a test person (324);
convert (202) the test result of each test into a test score having a common unit, wherein the conversion is based on a test-specific conversion model (400, 500); and
determine (204) a daily energy reserve for the test person (324) in time domain on the basis of the plurality of test scores, wherein the daily energy reserve indicates time duration the test person (324) can perform physical and/or mental activities above a predetermined intensity threshold during a day.
